(19) Europäisches Patentamt
European Patent Office
Office européen des brevets

(11) **EP 4 295 767 B1**

(12) **EUROPÄISCHE PATENTSCHRIFT**

(45) Veröffentlichungstag und Bekanntmachung des Hinweises auf die Patenterteilung:
**10.09.2025  Patentblatt 2025/37**

(21) Anmeldenummer: **22180128.5**

(22) Anmeldetag: **21.06.2022**

(51) Internationale Patentklassifikation (IPC):
*A61B 5/145* *(2006.01)*     *A61C 19/02* *(2006.01)*
*A61B 5/01* *(2006.01)*      *A61B 5/03* *(2006.01)*

(52) Gemeinsame Patentklassifikation (CPC):
**A61B 5/14507; A61B 5/0002; A61B 5/14546; A61B 5/682; A61C 19/02;** A61B 5/01; A61B 5/038; A61B 5/14532; A61B 5/14539; A61B 5/4277; A61B 5/4845; A61B 2560/0214; A61B 2560/0257; A61B 2560/0406; A61B 2562/0219;     (Forts.)

(54) **SCHALTUNGSGEHÄUSE FÜR EINE TRAGBARE INTRAORALE ANWENDUNG**

CIRCUIT HOUSING FOR PORTABLE INTRAORAL APPLICATION

LOGEMENT DE CIRCUIT POUR UNE APPLICATION INTRABUCCALE PORTABLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**27.12.2023  Patentblatt 2023/52**

(73) Patentinhaber: **Ivoclar Vivadent AG 9494 Schaan (LI)**

(72) Erfinder:
• **RIST, Kai**
  **6800 Feldkirch (AT)**
• **KUHN, Marvin**
  **6800 Feldkirch (AT)**
• **ALEXANDER, Skawran**
  **7320 Sargans (CH)**

(74) Vertreter: **Baldus, Oliver Splanemann Rumfordstrasse 7 80469 München (DE)**

(56) Entgegenhaltungen:
**US-A1- 2018 000 563     US-A1- 2022 183 623 US-A1- 2022 183 624**

(52) Gemeinsame Patentklassifikation (CPC): (Forts.)
A61B 2562/029; A61B 2562/164

**Beschreibung**

Die vorliegende Erfindung betrifft ein Schaltungsgehäuse für eine tragbare intraorale Anwendung und ein Verfahren zum Herstellen eines Schaltungsgehäuse für eine tragbare intraorale Anwendung.

Die Druckschrift US 4,629,424 A betrifft eine Vorrichtung zur intraoralen Umgebungserfassung, die eine abnehmbare orale Vorrichtung mit einer Anzahl von chemisch empfindlichen Elektroden und einer gemeinsamen Referenzelektrode an den chemischen Erfassungsstellen umfasst.

Die Druckschrift US 2018/000563 A1 betrifft Geräte zur Überwachung einer kieferorthopädischen Apparatur. Ein Material wird über dem Überwachungsgerät und dem Modell geformt, um ein Gerätegehäuse herzustellen.

Die Druckschrift US 2022/183623 A1 betrifft ein Dentalobjekt zum Befestigen an einem Zahn oder an Zähnen und ein Verfahren zum Befestigen eines Dentalobjekts an einem Zahn oder an Zähnen. Das Dentalobjekt weist einen Haftbereich auf, der Gutta-Percha-Material zum Befestigen des Dentalobjekts an dem Zahn umfasst.

Die Druckschrift US 2022/183624 A1 betrifft einen Dentalsensor für einen Intraoralbereich und ein Verfahren zum Einsetzen eines Dentalsensors.

Zurzeit werden elektronische Geräte für einen Intraoralraum durch ein äußeres, separates Gehäuse aus Polymeren geschützt. Neue Materialien erlauben dagegen kleinere Baugrößen von elektronischen Geräten für den dentalen Bereich. Diese Gehäuse werden oft undicht und schützen die innenliegende Elektronik nur unzureichend.

Es ist die technische Aufgabe der vorliegenden Erfindung, ein Schaltungsgehäuse bereitzustellen, mit dem eine elektronische Schaltung in einem Intraoralraum besser vor Feuchtigkeit geschützt werden kann.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Schaltungsgehäuse für eine tragbare intraorale Anwendung gelöst, mit einer inneren Schicht aus einem ersten Material zum wasserdichten Einschließen einer elektronischen Schaltung, wobei das erste Material der inneren Schicht mindestens einen Schutzlack umfasst, der aus Silikon, (Meth)acrylatharz, Urethanharz, Epoxidharz, Parylene oder Mischungen davon besteht; und einer äußeren Schicht aus einem zweiten Material zum räumlichen Anpassen des Schaltungsgehäuses an ein Gebiss, die das erste Material zumindest teilweise abdeckt. Durch den Schutzlack wird beispielsweise der technische Vorteil erreicht, dass besonders geeignete Materialien zum wasserdichten Einschließen der elektronischen Schaltung verwendet werden.

Durch das Schaltungsgehäuse wird der technische Vorteil erreicht, dass Wassereinbrüche in das Schaltungsgehäuse aufgrund einer Kaubewegung verhindert werden können. Zudem dient die zweite Schicht zum mechanischen Schutz vor Beschädigung der ersten Schicht. Das Schaltungsgehäuse mit zwei separaten Schichten ist robuster und weniger anfällig für Wassereinbrüche. Durch das Schaltungsgehäuse können elektronische Schaltungen für den medizinischen und dentalen Bereich vor äußeren Einflüssen wie Wasser, Speichel, anderen Flüssigkeiten oder Verunreinigungen geschützt werden und ein Ausfall verhindert werden.

In einer weiteren technisch vorteilhaften Ausführungsform des Schaltungsgehäuses umfasst das zweite Material der äußeren Schicht mindestens ein 3D-druckbares Material (beispielweise bei Stereolithographie), insbesondere lichtpolymerisierbare Harze, vorzugsweise (Meth)acrylate, Epoxidharze, Urethanharze, Thermoplaste, und/oder das zweite Material umfasst ein fräsbares Material, insbesondere Poly(meth)acrylat, Polycarbonat oder Keramik. Das zweite Material kann aus einem aus einem Material gebildet sein, das in einem dreidimensionalen Druckverfahren oder Fräsverfahren verwendet werden kann. Dadurch wird beispielsweise der technische Vorteil erreicht, dass das Schaltungsgehäuse auf einfache Weise räumlich an unterschiedliche Intraoralräume angepasst werden kann.

In bevorzugten Ausführungsformen, Materialien für das zweite Material der äußeren Schicht sind orthodontische Schienen (z.B. Aufbissschienen) Materialien wie ProArt Print Splint der Firma Ivoclar Vivadent.

In einer weiteren technisch vorteilhaften Ausführungsform des Schaltungsgehäuses weist das zweite Material eine Bruchzähigkeit von mehr als 0,5 $MPa\sqrt{m}$ oder ein Biegemodul von weniger als 2500 MPa auf. Dadurch wird beispielsweise der technische Vorteil erreicht, dass sich das zweite Material gut an ein Gebiss anpasst.

In einer weiteren technisch vorteilhaften Ausführungsform des Schaltungsgehäuses lässt das erste und das zweite Material eine Transmission elektromagnetischer Strahlung im Bereich zwischen 2,2 und 2,4 GHz zu. Dadurch wird beispielsweise der technische Vorteil erreicht, dass Daten durch das Schaltungsgehäuse hindurch über Bluetooth übermittelt werden können.

In einer weiteren technisch vorteilhaften Ausführungsform des Schaltungsgehäuses lassen das erste und das zweite Material eine Transmission elektromagnetischer Strahlung im Bereich zwischen 13 und 14 MHz zu. Dadurch wird beispielsweise der technische Vorteil erreicht, dass Daten durch das Schaltungsgehäuse hindurch über Nahfeldkommunikation (Near Field Communication - NFC) übertragen werden können.

In einer weiteren technisch vorteilhaften Ausführungsform des Schaltungsgehäuses weisen das erste und das zweite Material eine elektrische Leitfähigkeit unter 10$^{-10}$ S/m auf. Dadurch wird beispielsweise der technische Vorteil erreicht, dass Verlustströme verhindert werden können.

In einer weiteren technisch vorteilhaften Ausführungsform des Schaltungsgehäuses umfasst das Schaltungsgehäuse eine Öffnung für einen Sensor. Dadurch wird beispielsweise der technische Vorteil erreicht, dass Speichel zur Messung an den Sensor herangeführt werden kann.

In einer weiteren technisch vorteilhaften Ausführungsform des Schaltungsgehäuses umfasst das Schaltungsgehäuse eine elektronische Schaltung mit einer Vorrichtung mit einer Anzahl von chemisch, biologisch und/oder physikalischen Sensoren. Beispiele sind eine Schaltung mit einem oder mehreren pH-Sensor, einem Laktatsensor, einem Temperatursensor, einem Glukosesensor, einen Sensor für flüchtige Schwefelkomponenten, einem Alkoholsensor, einem Luftdrucksensor, einem Cortisolsensor, einem Osmolalitätssensor, einem ionenselektiven Sensor, einem Beschleunigungssensor, einem Drucksensor und/oder einem Feuchtigkeitssensor. Dadurch wird beispielsweise der technische Vorteil erreicht, dass besonders geeignete Sensoren für einen Intraoralraum verwendet werden. Das Schaltungsgehäuse kann auch mehrere Sensoren umfassen. Es können beispielsweise bei einer Laktat- oder pH-Wertmessung auch zwei gleiche Sensoren in einer Schaltung genutzt werden, um Messwerte an verschiedenen Stellen zu generieren oder zu einer Kontrolle des ersten Sensors.

In einer technisch vorteilhaften Ausführungsform einer Schaltungsgehäuseanordnung umfasst die Schaltungsgehäuseanordnung das Schaltungsgehäuse und eine elektronische Schaltung mit einer Vorrichtung mit einer Anzahl von chemisch, biologisch und/oder physikalischen Sensoren. Beispiele sind eine Schaltung mit einem oder mehreren pH-Sensor, einem Laktatsensor, einem Temperatursensor, einem Glukosesensor, einen Sensor für flüchtige Schwefelkomponenten, einem Alkoholsensor, einem Luftdrucksensor, einem Cortisolsensor, einem Osmolalitätssensor, einem ionenselektiven Sensor, einem Beschleunigungssensor, einem Drucksensor und/oder einem Feuchtigkeitssensor. Dadurch wird beispielsweise der technische Vorteil erreicht, dass besonders geeignete Sensoren für einen Intraoralraum verwendet werden. Die Schaltungsgehäuseanordnung kann auch mehrere Sensoren umfassen. Es können beispielsweise bei einer Laktat- oder pH-Wertmessung auch zwei gleiche Sensoren in einer Schaltung genutzt werden, um Messwerte an verschiedenen Stellen zu generieren oder zu einer Kontrolle des ersten Sensors.

In einer weiteren technisch vorteilhaften Ausführungsform des Schaltungsgehäuses umfasst das Schaltungsgehäuse eine elektronische Schaltung mit einem Schaltungsabschnitt zum drahtlosen Übertragen von Energie, eine Sende-/Empfangseinheit für eine drahtlose Datenübertragung und/oder einen Energiespeicher. Dadurch wird beispielsweise der technische Vorteil erreicht, dass Energie und/oder Daten an die elektronische Schaltung übertragen werden können.

In einer weiteren technisch vorteilhaften Ausführungsform einer Schaltungsgehäuseanordnung umfasst die Schaltungsgehäuseanordnung das Schaltungsgehäuse und eine elektronische Schaltung mit einem Schaltungsabschnitt zum drahtlosen Übertragen von Energie, eine Sende-/Empfangseinheit für eine drahtlose Datenübertragung und/oder einen Energiespeicher. Dadurch wird beispielsweise der technische Vorteil erreicht, dass Energie und/oder Daten an die elektronische Schaltung übertragen werden können.

In einer weiteren technisch vorteilhaften Ausführungsform des Schaltungsgehäuses umfasst das Schaltungsgehäuse eine Zwischenschicht zum Verbinden der inneren Schicht und der äußeren Schicht. Dadurch wird beispielsweise der technische Vorteil erreicht, dass die Haftung zwischen der inneren und der äußeren Schicht verbessert werden kann oder ein mechanischer Schutz der inneren Komponenten erhöht wird.

In einer weiteren technisch vorteilhaften Ausführungsform des Schaltungsgehäuses umfasst das Schaltungsgehäuse eine elektronische Schaltung, die in das erste Material eingegossen ist. Dadurch wird beispielsweise der technische Vorteil erreicht, dass ein Feuchtigkeitsschutz weiter verbessert wird.

In einer weiteren technisch vorteilhaften Ausführungsform einer Schaltungsgehäuseanordnung umfasst die Schaltungsgehäuseanordnung das Schaltungsgehäuse und eine elektronische Schaltung, die in das erste Material eingegossen ist. Dadurch wird beispielsweise der technische Vorteil erreicht, dass ein Feuchtigkeitsschutz weiter verbessert wird.

Gemäß einem zweiten Aspekt wird die technische Aufgabe durch ein Verfahren zum Herstellen eines Schaltungsgehäuse für eine tragbare intraorale Anwendung gelöst, mit den Schritten eines Anordnens einer inneren Schicht aus einem ersten Material zum wasserdichten Einschließen einer elektronischen Schaltung, wobei das erste Material der inneren Schicht mindestens einen Schutzlack umfasst, der aus Silikon, (Meth)acrylatharz, Urethanharz, Epoxidharz, Parylene oder Mischungen davon besteht; und eines Anordnens einer äußeren Schicht aus einem zweiten Material zum räumlichen Anpassen des Schaltungsgehäuses an ein Gebiss, die das erste Material zumindest teilweise abdeckt. Dadurch werden die gleichen technischen Vorteile wie durch das Schaltungsgehäuse nach dem ersten Aspekt erreicht.

In einer technisch vorteilhaften Ausführungsform des Verfahrens wird die elektronische Schaltung in das erste Material eingegossen. Dadurch wird beispielsweise der technische Vorteil erreicht, dass ein Eindringen von Feuchtigkeit besonders wirksam verhindert wird.

In einer weiteren technisch vorteilhaften Ausführungsform des Verfahrens wird das erste Material auf die elektronische Schaltung auflackiert oder durch physikalische Gasphasenabscheidung oder chemische Gasphasenabscheidung angebracht. Dadurch wird beispielsweise der technische Vorteil erreicht, dass die elektronische Schaltung mit einem dünnen Film besonders wasserfest eingeschlossen werden kann.

Ausführungsbeispiele sind in den Zeichnungen dargestellt und werden im Folgenden näher beschrieben.

Es zeigen:

Fig. 1 eine schematische Abbildung eines Schaltungsgehäuses in einer ersten Ausführungsform;

Fig. 2 eine schematische Abbildung eines Schaltungsgehäuses in einer zweiten Ausführungsform; und

Fig. 3 ein Blockdiagramm eines Verfahrens zum Herstellen eines Schaltungsgehäuses.

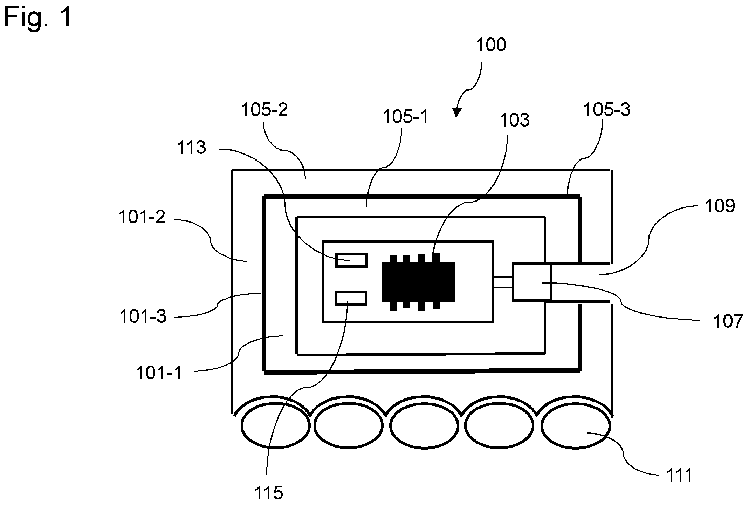

Fig. 1 zeigt eine schematische Abbildung eines Schaltungsgehäuses 100 für eine tragbare intraorale elektronische Schaltung 103. Das Schaltungsgehäuse 100 kann in einem Mund eines Patienten als tragbare Vorrichtung (Wearable) verwendet werden.

Die verwendete elektronische Schaltung 103 innerhalb des Schaltungsgehäuses 100 wird intraoral über einen Zeitraum von mehreren Minuten bis Tagen getragen. Das Schaltungsgehäuse 100 schützt die elektronische Schaltung 103 hierbei vor dem Speichel und aufgenommener Nahrung oder Flüssigkeit. Zudem schützt das Schaltungsgehäuse 100 die elektronische Schaltung 103 vor einer Beschädigung durch versehentliches Beißen. Das Schaltungsgehäuse 100 stellt somit eine Versiegelung für die elektronische Schaltung 103 vor äußeren Einflüssen bereit. Für die Versiegelung können Materialien verwendet werden, die im intraoralen Bereich bereits zugelassen sind. Da das Schaltungsgehäuse 100 in einem Mund getragen wird, können hierzu biokompatible Materialien verwendet werden. Für die Bewertung der Biokompatibilität von Dentalmaterialien wird die Norm EN ISO 7405:2019 verwendet.

Der Schutz der elektronischen Schaltung 103 folgt einem Zwiebelprinzip. Das Schaltungsgehäuse 100 umfasst eine innere Schicht 105-1 aus einem biokompatiblen Material 101-1 zum wasserdichten Einschließen einer elektronischen Schaltung 103.

Auf der inneren Schicht befindet sich eine äußere Schicht 105-2 aus einem biokompatiblen Material 101-2 zum räumlichen Anpassen des Schaltungsgehäuses an ein Gebiss 111. Die äußere Schicht deckt das erste Material 101-1 der inneren Schicht 105-1 zumindest teilweise ab und umgibt dabei das erste Material 101-1.

Die äußere Schicht 105-2 kann patientenindividuell gefertigt sein und sich nahtlos an das Gebiss 111 anpassen, so dass diese einem äußeren Druck beim Zusammenbeißen von Zähnen widersteht. Zur Herstellung der äußeren Schicht 105-2 sind daher formgebende additive oder subtraktive Fertigungsverfahren vorteilhaft, da diese die äußere Schicht 105-1 in der gewünschten räumlichen Form herstellen können. Hierzu bieten sich dreidimensionale Druck- oder Spritzgussverfahren an.

Das Material 101-1 der inneren Schicht 105-1 ist beispielsweise durch einen Schutzlack (Conformal Coating) gebildet. Der Schutzlack kann aus Silikon, (Meth)Acrylatharz, Urethanharz, Epoxidharz, Parylene oder deren Mischungen bestehen. Das Material 101-1 der inneren Schicht 101-5 kann auch Polyvinylsiloxan umfassen. In bevorzugten Ausführungsformen, Materialien für das Material 101-1 der inneren Schicht 105-1 sind Polyvinylsiloxanabformmaterialien wie die der Virtual-Produktlinie der Firma Ivoclar Vivadent Putty, Heavy Body, Monophase, Light Body und Extra Light Body, oder (Meth)acrylatharze wie Heliobond der Firma Ivoclar Vivadent.

Die innere Schicht 105-1 sollte möglichst dünn sein. Die innere Schicht 105-1 weist beispielsweise eine minimale Dicke von vorzugsweise 5 mm bis 0.0001 mm auf, ganz bevorzugt 3 mm bis 0.001, besonders bevorzugt 1 mm bis 0.01 mm. Die innere Schicht 105-1 weist beispielsweise eine maximale Dicke von vorzugsweise 10 mm bis 0.0001 mm auf, ganz bevorzugt 6 mm bis 0.001, besonders bevorzugt 2 mm bis 0.01 mm. Die äußere Schicht 105-2 weist beispielsweise eine minimale Dicke von vorzugsweise 15 mm bis 0.1 mm auf, ganz bevorzugt 12 mm bis 0.5 mm, besonders bevorzugt 10 mm bis 1 mm. Die äußere Schicht 105-2 weist beispielsweise eine maximale Dicke von vorzugsweise 30 mm bis 0.1 mm auf, ganz bevorzugt 20 mm bis 0.5 mm, besonders bevorzugt 15 mm bis 1 mm.

Das Material 101-2 der äußeren Schicht 105-2 umfasst ein Material, das in einem dreidimensionalen Druckverfahren verwendet werden kann, wie beispielsweise eine radikalisch polymerisierbare Zusammensetzung oder ein lichtpolymerisierbares Harz für die Stereolithografie. Das Material 101-2 der äußeren Schicht 105-2 kann mindestens ein 3D-druckbares Material umfassen, insbesondere lichtpolymerisierbare Harze, vorzugsweise (Meth)acrylate, Epoxidharze, Urethanharze, und/oder Thermoplaste. Das zweite Material 101-2 kann ebenfalls ein fräsbares Material umfassen, insbesondere Poly(meth)acrylat, Polycarbonat oder Keramik.

Das Schaltungsgehäuse 100 kann zudem eine Zwischenschicht 101-3 als Bindematerial zwischen der inneren Schicht 101-1 und der äußeren Schicht 101-2 umfassen. Das Material 105-3 der Zwischenschicht 101-3 dient als Bindematerial und ist ein Vergussmaterial oder eine Vergussmasse (Potting Material), wie beispielsweise Silikon, Urethanharz, Epoxidharz oder (Meth)acrylatharz.

Die elektronische Schaltung 103 umfasst eine Sende-/Empfangseinheit 113 für die kabellose Datenübertragung, wie beispielsweise eine Kommunikationseinheit für die kabellose Datenübertragung wie Bluetooth in einem oder NFC. Um diese Datenübertragung zu ermöglichen, sind die Materialien 101-1, 1012 und 101-3 des Schaltungsgehäuses 100 für eine elektromagnetische Strahlung im Bereich zwischen 2,2 und 2,4 GHz (Bluetooth) oder im Bereich zwischen 13 und 14 MHz (NFC) durchlässig. Zudem kann die elektronische Schaltung 103 einen kabellos aufladbaren Akku als Energiespeicher 115 umfassen. Zu diesem Zweck verfügt die elektronische Schaltung 103 einen Schaltungsabschnitt zum drahtlosen Übertragen von Energie, beispielsweise mit einer Induktionsschleife. Die verwendeten Materialien 101-1, 101-2 und 101-3 erlauben daher das kabellose Laden und die Kommunikation per Bluetooth oder NFC.

Das Schaltungsgehäuse 100 kann elektrische Ausgänge oder Leitungen für unterschiedliche Sensoren 107 der elektronischen Schaltung 103 enthalten. Die Sensoren 107 dienen beispielsweise zur Bestimmung der Menge verschiedener Stoffe, wie beispielsweise Laktat, Glukose, Cortisol, Alkohol oder flüchtige Schwefelkomponenten (Volatile Sulphur Compounds - VSC). Die Sensoren 107 können zudem zur Messung eines pH-Wertes, einer Temperatur, eines Luftdrucks oder einer Osmolalität vorgesehen sein.

Um diese Stoffe und Werte messen zu können, sind die Sensoren 107 in direkten Kontakt mit dem Speichel. Je nach Anwendung sind die Sensoren 107 nicht ummantelt, damit der Speichel oder die Luft mit dem Sensor 107 in Kontakt kommen kann. Zu diesem Zweck umfasst das Schaltungsgehäuse 100 zumindest eine Öffnung 109, die sich durch die Schichten 101-1, 101-2 und 101-3 hindurch erstreckt und die Flüssigkeit an den Sensor 107 heranführt. Die Öffnungen 109 der unterschiedlichen Sensoren 107 können sich in Form, Größe und Position unterscheiden und an das individuelle Schaltungsgehäuse 100 des Trägers angepasst werden.

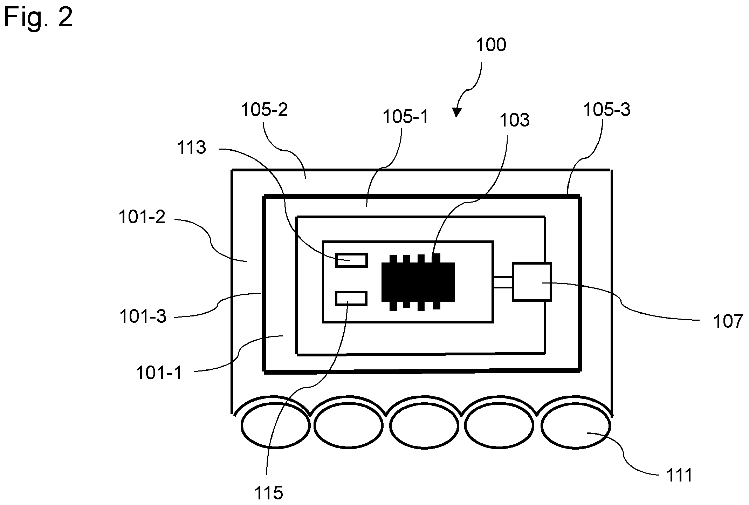

Fig. 2 zeigt eine schematische Abbildung eines Schaltungsgehäuses 100 in einer zweiten Ausführungsform. In dieser Ausführungsform sind die innere Schicht 105-1, die Zwischenschicht 105-3 und die äußere Schicht 105-2 vollständig geschlossen um die elektronische Schaltung 103 herum aufgebaut. Das Schaltungsgehäuse 100 weist demnach keine Öffnung 109 auf.

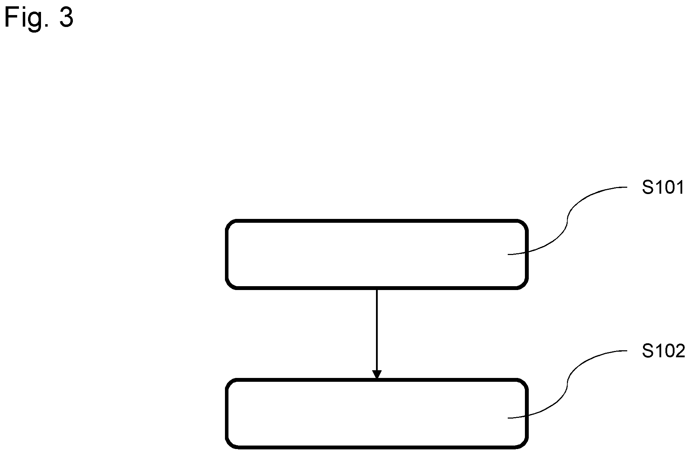

Fig. 3 zeigt ein Blockdiagramm eines Verfahrens zum Herstellen eines Schaltungsgehäuses 100 für eine tragbare intraorale Anwendung. In einem ersten Schritt S101 wird eine innere Schicht aus dem ersten Material 101-1 zum wasserdichten Einschließen der elektronischen Schaltung 103 angeordnet. Die elektronische Schaltung 103 wird beispielsweise in Silikon oder ein anderes Material eingegossen, um diese wasserdicht zu halten. Diese Ummantelung bildet die erste Schutzschicht zum Schutze der elektronischen Schaltung 103.

In einem weiteren Schritt S102 wird die äußere Schicht aus dem zweiten Material 101-2 zum räumlichen Anpassen des Schaltungsgehäuses an das Gebiss 111 angeordnet, das das erste Material 101-1 zumindest teilweise abdeckt.

Die innere Schicht kann beispielsweise auflackiert werden. Die innere Schicht 105-1 wird vorzugsweise nicht gedruckt, sondern durch Umhüllen/ Verkapselung wie Vergießen, Potting, Tauchbeschichtung (Dip Coating), Sprühbeschichtung (Spray Coating), Beschichtung mittels Pinsel (Brush Coating), mittels physikalischer Gasphasenabscheidung (Physical Vapour Deposition - PVD) oder chemischer Gasphasenabscheidung (Chemical Vapour Deposition - CVD) auf die elektronische Schaltung 103 aufgebracht. Im Allgemeinen kann die innere Schicht 105-1 thermisch, chemisch oder durch UV-Strahlung oder Lufttrocknung ausgehärtet werden. Diese Verfahren sind auf einfache Weise handhabbar und die elektronische Schaltung 103 kann mit einem dünnen Film als innerer Schicht 101-1 geschützt werden. Die elektronische Schaltung 103 kann je nach Bedarf nur teilweise oder vollständig versiegelt werden.

Je nach Größe der elektronischen Schaltung 103 kann diese auch in das Material 101-1 eingetaucht oder mit diesem lackiert oder ummantelt werden. Nach dem Aushärten des Materials 101-1 ist die elektronische Schaltung 103 von einer schützenden Schicht umgeben. Es können auch mehrere Schichten aus dem Material 101-1 für eine Versiegelung aufgetragen werden. Nach einer vollständigen Versiegelung ist die elektronische Schaltung 103 wasserdicht eingeschlossen.

Nach dem ersten Schritt S101 kann auch zunächst eine flexible und verbindende Zwischenschicht 101-3, wie beispielsweise ein Kleber, zum Verbinden der inneren Schicht 101-1 mit der äußeren Schicht 101-2 aufgebracht werden. Die Zwischenschicht 101-3 bildet zudem einen weiteren mechanischen Schutz für die darunterliegende Schicht 101-1 und die elektronische Schaltung. Die Zwischenschicht 101-3 kann aus einem dritten Material als Bindematerial gebildet sein, wie beispielsweise Polyvinylether oder Silikon, insbesondere Additionssilikon oder Vinylpolysiloxan.

In einer dentalen Anwendung kann die versiegelte elektronische Schaltung 103 schadlos Speichel und anderen Flüssigkeiten im Mund widerstehen. Die Materialien 101-1, 101-2 und 101-3 können von der elektronische Schaltung 103 bei Bedarf wieder entfernt werden.

Alle in Verbindung mit einzelnen Ausführungsformen erläuterten und gezeigten Merkmale können in unterschiedlicher Kombination vorgesehen sein, um gleichzeitig deren vorteilhafte Wirkungen zu realisieren. Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

**Patentansprüche**

1. Schaltungsgehäuse (100) für eine tragbare intraorale Anwendung, mit:

   - einer inneren Schicht (105-1) aus einem ersten Material (101-1) zum wasserdichten Einschließen einer elektronischen Schaltung (103), wobei das erste Material (101-1) mindestens einen Schutzlack umfasst, der aus Silikon, (Meth)acrylatharz, Urethanharz, Epoxidharz, Parylene oder Mischungen davon besteht; und
   - einer äußeren Schicht (105-2) aus einem zweiten Material (101-2) zum räumlichen Anpassen des Schaltungsgehäuses an ein Gebiss (111), die das erste Material (101-1) zumindest teilweise abdeckt.

2. Schaltungsgehäuse (100) nach Anspruch 1, wobei das zweite Material (101-2) mindestens ein 3D-druckbares Material umfasst, insbesondere lichtpolymerisierbares Harz, vorzugsweise (Meth)acrylatharz, Epoxidharz, Urethanharz, Thermoplast, und/oder das zweite Material (101-2) ein fräsbares Material umfasst, insbesondere Poly(meth)acrylat, Polycarbonat oder Keramik.

3. Schaltungsgehäuse (100) nach einem der vorangehenden Ansprüche, wobei das zweite Material (101-2) eine Bruchzähigkeit von mehr als 0,5 $MPa\sqrt{m}$ oder ein Biegemodul von weniger als 2500 MPa aufweist.

4. Schaltungsgehäuse (100) nach einem der vorangehenden Ansprüche, wobei das erste und das zweite Material (101-1, 101-2) eine Transmission elektromagnetischer Strahlung im Bereich zwischen 2,2 und 2,4 GHz zulassen.

5. Schaltungsgehäuse (100) nach einem der vorangehenden Ansprüche, wobei das erste und das zweite Material (101-1, 101-2) eine Transmission elektromagnetischer Strahlung im Bereich zwischen 13 und 14 MHz zulassen.

6. Schaltungsgehäuse (100) nach einem der vorangehenden Ansprüche, wobei das erste und das zweite Material (101-1, 101-2) eine elektrische Leitfähigkeit unter $10^{-10}$ S/m aufweisen.

7. Schaltungsgehäuse (100) nach einem der vorangehenden Ansprüche, wobei das Schaltungsgehäuse (100) eine Öffnung (109) für einen Sensor (107) umfasst.

8. Schaltungsgehäuse (100) nach einem der vorangehenden Ansprüche, wobei das Schaltungsgehäuse (100) eine elektronische Schaltung (103) mit einem pH-Sensor, einem Laktatsensor, einem Temperatursensor, einem Glukosesensor, einen Sensor für flüchtige Schwefelkomponenten, einem Alkoholsensor, einem Luftdrucksensor, einem Cortisolsensor, einem Osmolalitätssensor, einem ionenselektiven Sensor, einem Beschleunigungssensor, einem Drucksensor und/oder einem Feuchtigkeitssensor umfasst.

9. Schaltungsgehäuse (100) nach einem der vorangehenden Ansprüche, wobei das Schaltungsgehäuse (100) eine elektronische Schaltung (103) zum drahtlosen Übertragen von Energie, eine Sende-/Empfangseinheit (113) für eine drahtlose Datenübertragung und/oder einen Energiespeicher (115) umfasst.

10. Schaltungsgehäuse (100) nach einem der vorangehenden Ansprüche, wobei das Schaltungsgehäuse (100) eine Zwischenschicht (101-3) zum Verbinden der inneren Schicht (101-1) und der äußeren Schicht (101-2) umfasst.

11. Schaltungsgehäuse (100) nach einem der vorangehenden Ansprüche, wobei das Schaltungsgehäuse (100) eine elektronische Schaltung (103) umfasst, die in das erste Material (101-1) eingeschlossen ist.

12. Verfahren zum Herstellen eines Schaltungsgehäuses (100) für eine tragbare intraorale Anwendung, mit den Schritten:

   - Anordnen (S101) einer inneren Schicht aus einem ersten Material (101-1) zum wasserdichten Einschließen einer elektronischen Schaltung (103), wobei das erste Material (101-1) mindestens einen Schutzlack umfasst, der aus Silikon, (Meth)acrylatharz, Urethanharz, Epoxidharz, Parylene oder Mischungen davon besteht; und
   - Anordnen (S102) einer äußeren Schicht aus einem zweiten Material (101-2) zum räumlichen Anpassen des Schaltungsgehäuses an ein Gebiss (111), die das erste Material (101-1) zumindest teilweise abdeckt.

13. Verfahren nach Anspruch 12, wobei die elektronische Schaltung (103) in das erste Material (101-1) eingeschlossen wird.

14. Verfahren nach Anspruch 12 oder 13, wobei das erste Material (101-1) auf die elektronische Schaltung (103) auflackiert wird.

## Claims

1. A circuit housing (100) for a wearable intraoral application, comprising:

   - an inner layer (105-1) of a first material (101-1) for waterproof enclosure of an electronic circuit (103), wherein the first material (101-1) comprises at least one protective varnish consisting of silicone, (meth)acrylate resin, urethane resin, epoxy resin, parylene, or mixtures thereof; and
   - an outer layer (105-2) of a second material (101-2) for spatially fitting the circuit housing to a set of teeth (111), which at least partially covers the first material (101-1).

2. The circuit housing (100) according to claim 1, wherein the second material (101-2) comprises at least one 3D-printable material, in particular light-polymerizable resin, preferably (meth)acrylate resin, epoxy resin, urethane resin, thermoplastic, and/or the second material (101-2) comprises a millable material, in particular poly(meth)acrylate, polycarbonate or ceramic.

3. The circuit housing (100) according to any one of the preceding claims, wherein the second material (101-2) has a fracture toughness of greater than 0.5 $\mathrm{MPa}\sqrt{\mathrm{m}}$ or a flexural modulus of less than 2500 MPa.

4. The circuit housing (100) according to any one of the preceding claims, wherein the first and second materials (101-1, 101-2) allow transmission of electromagnetic radiation in the range between 2.2 and 2.4 GHz.

5. The circuit housing (100) according to any one of the preceding claims, wherein the first and second materials (101-1, 101-2) allow transmission of electromagnetic radiation in the range between 13 and 14 MHz.

6. The circuit housing (100) according to any one of the preceding claims, wherein the first and second materials (101-1, 101-2) have an electrical conductivity below $10^{-10}$ S/m.

7. The circuit housing (100) according to any one of the preceding claims, wherein the circuit housing (100) comprises an opening (109) for a sensor (107).

8. The circuit housing (100) according to any one of the preceding claims, wherein the circuit housing (100) comprises an electronic circuit (103) having a pH sensor, a lactate sensor, a temperature sensor, a glucose sensor, a volatile sulfur compound sensor, an alcohol sensor, an atmospheric pressure sensor, a cortisol sensor, an osmolality sensor, an ion-selective sensor, an acceleration sensor, a pressure sensor, and/or a moisture sensor.

9. The circuit housing (100) according to any one of the preceding claims, wherein the circuit housing (100) comprises an electronic circuit (103) for wireless transmission of energy, a transceiver unit (113) for wireless data transmission and/or an energy storage (115).

10. The circuit housing (100) according to any one of the preceding claims, wherein the circuit housing (100) comprises an intermediate layer (101-3) for connecting the inner layer (101-1) and the outer layer (101-2).

11. The circuit housing (100) according to any one of the preceding claims, wherein the circuit housing (100) comprises an electronic circuit (103) enclosed in the first material (101-1).

12. A method of manufacturing a circuit housing (100) for a wearable intraoral application, comprising the steps of:

    - arranging (S101) an inner layer of a first material (101-1) for waterproof enclosure of an electronic circuit (103), wherein the first material (101-1) comprises at least one protective varnish consisting of silicone, (meth)acrylate resin, urethane resin, epoxy resin, parylene, or mixtures thereof; and
    - arranging (S102) an outer layer of a second material (101-2) for spatially fitting the circuit housing to a set of teeth (111), which at least partially covers the first material (101-1).

13. The method according to claim 12, wherein the electronic circuit (103) is enclosed in the first material (101-1).

14. The method according to claim 12 or 13, wherein the first material (101-1) is varnished onto the electronic circuit (103).

## Revendications

1. Boîtier de circuit (100) destiné à une application intra-orale portable, comprenant :

   - une couche interne (105-1) constituée d'un premier matériau (101-1) pour enfermer de manière étanche un circuit électronique (103), où le premier matériau (101-1) comprend au moins un vernis protecteur constitué de silicone, de résine (méth)acrylate, de résine uréthane, de résine époxy, de parylène ou de mélanges de ceux-ci ; et

- une couche externe (105-2) constituée d'un deuxième matériau (101-2) destiné à adapter spatialement le boîtier de circuit à une dentition (111), qui recouvre au moins partiellement le premier matériau (101-1).

2. Boîtier de circuit (100) selon la revendication 1, où le deuxième matériau (101-2) comprend au moins un matériau imprimable en 3D, en particulier une résine photopolymérisable, de préférence une résine (méth)acrylate, une résine époxy, une résine uréthane, thermoplastique, et/ou le deuxième matériau (101-2) comprend un matériau pouvant être fraisé, en particulier du poly(méth)acrylate, du polycarbonate ou de la céramique.

3. Boîtier de circuit (100) selon l'une des revendications précédentes, où le deuxième matériau (101-2) présente une ténacité à la rupture supérieure à 0,5 MPa√m ou un module de flexion inférieur à 2500 MPa.

4. Boîtier de circuit (100) selon l'une des revendications précédentes, où le premier et le deuxième matériau (101-1, 101-2) permettent une transmission du rayonnement électromagnétique dans la plage comprise entre 2,2 et 2,4 GHz.

5. Boîtier de circuit (100) selon l'une des revendications précédentes, où le premier et le deuxième matériau (101-1, 101-2) permettent la transmission d'un rayonnement électromagnétique dans la gamme comprise entre 13 et 14 MHz.

6. Boîtier de circuit (100) selon l'une des revendications précédentes, où le premier et le deuxième matériau (101-1, 101-2) présentent une conductivité électrique inférieure à $10^{-10}$ S/m.

7. Boîtier de circuit (100) selon l'une des revendications précédentes, où le boîtier de circuit (100) comprend une ouverture (109) pour un capteur (107).

8. Boîtier de circuit (100) selon l'une des revendications précédentes, où le boîtier de circuit (100) comprend un circuit électronique (103) avec un capteur de pH, un capteur de lactate, un capteur de température, un capteur de glucose, un capteur de composants soufrés volatils, un capteur d'alcool, un capteur de pression atmosphérique, un capteur de cortisol, un capteur d'osmolalité, un capteur sélectif d'ions, un capteur d'accélération, un capteur de pression et/ou un capteur d'humidité.

9. Boîtier de circuit (100) selon l'une des revendications précédentes, où le boîtier de circuit (100) comprend un circuit électronique (103) pour la transmission sans fil d'énergie, une unité d'émission/réception (113) pour la transmission sans fil de données et/ou un stockage d'énergie (115).

10. Boîtier de circuit (100) selon l'une des revendications précédentes, où le boîtier de circuit (100) comprend une couche intermédiaire (101-3) pour relier la couche intérieure (101-1) et la couche extérieure (101-2).

11. Boîtier de circuit (100) selon l'une des revendications précédentes, où le boîtier de circuit (100) comprend un circuit électronique (103) enfermé dans le premier matériau (101-1).

12. Procédé de fabrication d'un boîtier de circuit (100) pour une application intra-orale portable, comprenant les étapes suivantes :

- disposition (S101) d'une couche interne d'un premier matériau (101-1) pour enfermer de manière étanche un circuit électronique (103), où le premier matériau (101-1) comprend au moins un vernis protecteur constitué de silicone, de résine (méth)acrylate, de résine uréthane, de résine époxy, de parylène ou de mélanges de ceux-ci ; et
- disposition (S102) d'une couche extérieure constituée d'un deuxième matériau (101-2) pour adapter spatialement le boîtier du circuit à une dentition (111), qui recouvre au moins partiellement le premier matériau (101-1).

13. Procédé selon la revendication 12, où le circuit électronique (103) est enfermé dans le premier matériau (101-1).

14. Procédé selon la revendication 12 ou 13, où le premier matériau (101-1) est appliqué par vernissage sur le circuit électronique (103).

Fig. 1

Fig. 2

Fig. 3

S101

S102

**IN DER BESCHREIBUNG AUFGEFÜHRTE DOKUMENTE**

**In der Beschreibung aufgeführte Patentdokumente**

- US 4629424 A
- US 2018000563 A1
- US 2022183623 A1
- US 2022183624 A1